# EUROPÄISCHE PATENTANMELDUNG

(11) **EP 0 552 645 A2**
(43) Veröffentlichungstag der Anmeldung: **28.07.1993**
(21) Anmeldenummer: 93100324.8
(22) Anmeldetag: 12.01.1993
(51) Int. Cl.: C07F 5/02, C07C 25/00, C07F 7/08, C07D 213/61

(54) **Verfahren zur Umsetzung von chlorierten Aromaten mit Elektrophilen**

(30) Priorität: 20.01.1992 DE 4201308
(71) Anmelder: MERCK PATENT GmbH, D-64271 Darmstadt (DE)
(72) Erfinder: Riefling, Bernhard, Dr., W-6100 Darmstadt (DE); Hittich, Reinhard, Dr., W-6101 Modautal (DE)

(57) **Zusammenfassung**

Die Erfindung betrifft ein Verfahren zur Umsetzung von chlorierten Aromaten mit Elektrophilen in ortho-Stellung zum Chloratom, dadurch gekennzeichnet, daß man zu einem Gemisch aus dem chlorierten Aromaten und dem Elektrophil eine starke Base zugibt.

## Beschreibung

Die Erfindung betrifft ein Verfahren zur Umsetzung von chlorierten Aromaten mit Elektrophilen in ortho-Stellung zum Chloratom, wobei man zu einem Gemisch aus dem chlorierten Aromaten und dem Elektrophil eine starke Base zugibt.

Chlorierte Aromaten, welche in ortho-Stellung zum Cl-Atom substituiert sind, sind bedeutende Zwischenprodukte in der organischen industriellen Chemie. Entsprechend substituierte Derivate stellen insbesondere wertvolle Zwischenprodukte zur Synthese von hochveredelten Endprodukten bzw. selbst solche Endprodukte für die Electronic-Industrie, wie z.B. Flüssigkristalle, für den Pflanzenschutz, wie z.B. Pestizide oder zur Herstellung von pharmazeutisch hochwirksamen Substanzen, wie z.B. Dopamin-Rezeptor-Blocker, Antiemetika oder Antipsychotika, dar.

Die bisher beschriebenen Verfahren zur Herstellung dieser Verbindungen eignen sich nicht zur großtechnischen Produktion, sondern stellen Verfahren dar, die lediglich im Labormaßstab risikolos durchgeführt werden können.

In der EP-0 440 082 wird ein Verfahren zur Umsetzung von fluorierten Aromaten beschrieben, wobei man zu einem Gemisch aus den fluorierten Aromaten und einem Elektrophil eine starke Base gibt.

Weiterhin ist jedoch bekannt, z.B. von B. Langlois, G. Soula, Bull. Soc. Chim. France 1986 No. 6, 925-929, daß in ortho-Stellung deprotonierte Chlorarylverbindungen leicht in einer exotherme Reaktion Chlorid abspalten, was zu Arinen führt und zur Bildung unerwünschter Nebenprodukte Anlaß gibt.

Eine solche Synthese kann natürlich nur in kleinem Maßstab im Labor durchgeführt werden. Für größere Ansätze in Produktionsbetrieben kommt dieses Verfahren nicht in Frage, da beim Ausfall des Kühlmittels die Apparatur zur potentiellen Bombe wird.

Aufgrund neuerer Entwicklungen in der Electronic-Industrie ist ein erheblicher Bedarf an Flüssigkristallen entstanden, welche einen ein- oder mehrfach chlorierten 1,4-Phenylenrest, insbesondere einen 2,3-Dichlor- bzw. 2,6-Dichlor-1,4-phenylenrest aufweisen. Die Befriedigung dieses Bedarfs unter Anwendung der bisher bekannten Verfahren stellt eine unlösbare Aufgabe dar, da eine risikolose Durchführung dieser Tieftemperaturreaktion im großen Maßstab nicht gewährleistet ist. Aufgabe der vorliegenden Erfindung war es, ein Herstellungsverfahren für o-Chlorphenyl-Derivate zu finden, das die beschriebenen Nachteile der bisherigen Verfahren nicht aufweist und risikolos im großtechnischen Maßstab durchzuführen ist.

Es wurde nun gefunden, daß sich die gewünschte Reaktion überraschenderweise "sicher" machen läßt, wenn man den Zugabemodus der Reaktionspartner ändert: Bei Vorlegen des Chlorarylderivates und des Elektrophils in einem inerten Lösungsmittel und Zutropfen von Butyllithium oder einer anderen starken Base wird intermediär entstandene o-Chloraryllithium-Verbindung sofort in-situ vom Elektrophil abgefangen und kann sich nicht anreichern und somit zu gefährlichen Nebenreaktionen führen. Dies ist deshalb überraschend, weil Butyllithium und z.B. Lithiumdiisopropylamid selbst mit dem Elektrophil reagieren kann und somit nicht ohne weiteres mit dem Entstehen der o-Chlorphenyl-Derivate gerechnet werden konnte.

Gegenstand der Erfindung ist somit ein Verfahren zur Umsetzung von chlorierten Aromaten mit Elektrophilen in ortho-Stellung zum Chloratom, dadurch gekennzeichnet, daß man zu einem Gemisch aus dem chlorierten Aromaten und dem Elektrophil eine starke Base zugibt, insbesondere ein Verfahren zur Herstellung von chlorierten Aromaten der Formel I, 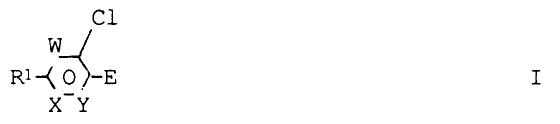
wobei
- R¹: H, F, Alkyl, Alkenyl, Alkoxy mit jeweils bis zu 18 C-Atomen oder eine mesogene Gruppe,
- W, X und Y: jeweils unabhängig voneinander N, CH oder Cl,
und
- E: -CH₂-R²-, 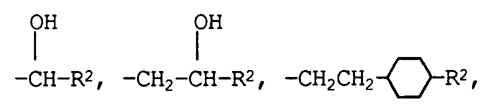 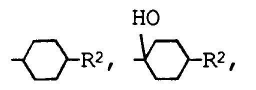 BX₂ oder SI,
worin
- R²: Alkyl mit 1 bis 15 C-Atomen oder einen der Gruppe R¹ entsprechender mesogenen Rest,
- BX₂: einen Trioxatriborinonrest der Formel 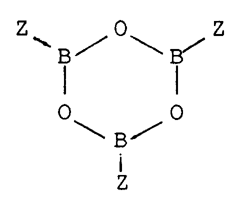 worin Z für 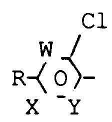 steht oder eine Gruppe der Formel -B(OR³)(OR⁴),
- R³ und R⁴: H, Alkyl, Alkenyl oder Cycloalkyl mit jeweils bis zu 10 C-Atomen oder zusammengenommen eine Alkylendiylgruppe der Formel -(CH₂)ₙ- oder -CH₂CHR⁸-CH₂-, worin n 2, 3 oder 4 ist und R⁸ Alkyl, Alkoxy oder Alkenyl mit bis zu 18 C-Atomen oder einen der Formel II entsprechenden mesogenen Rest, und
- SI: eine Trihydrocarbylsilylgruppe der Formel -Si(R⁵)₃ worin R⁵ jeweils unabhängig voneinander aliphatischer, cycloaliphatischer, araliphatischer oder aromatischer Rest ist,
bedeuten.

Insbesondere sind solche Verfahren Gegenstand der Erfindung, worin R¹ eine mesogene Gruppe der Formel II bedeutet, wobei

R⁰-A¹-Z¹-(-A²-Z²-)-ₘ II

- R^{O}: einen unsubstituierten oder einen einfach durch CN, Halogen oder CF₃ substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind,
- Z¹ und Z²: jeweils unabhängig voneinander -CH₂CH₂-, -C≡C-, -CH₂O-, -OCH₂-, -CO-O-, -O-CO-, -CH=N-, -N=CH-, -CH₂S-, -SCH₂-, eine Einfachbindung oder eine Alkylengruppe mit 3 bis 6 C-Atomen, worin auch eine CH₂-Gruppe durch -O-, -CO-O-, -O-CO-, -CHHalogen- oder -CHCN- ersetzt sein kann,
und
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,3-Cyclobutylen, 1,3-Bicyclo(1,1,1)pentylen, 1,4-Cyclohexenylen, 1,4-Bicyclo(2,2,2)octylen, Piperidin-1,4-diyl, Naphthalin-2,6-diyl, Decahydronaphthalin-2,6-diyl und 1,2, 3,4-Tetrahydronaphthalin-2,6-diyl,
wobei die Reste (a) und (b) durch CN oder Halogen substituiert sein können, und
- m: 0, 1 oder 2
bedeuten.

Bei der Umsetzung der chlorierten Aromaten mit den entsprechenden Boraten entstehen in der Regel zuerst die cyclischen Trimere der entsprechenden o-Chlorarylboronsäure der Formel IB' 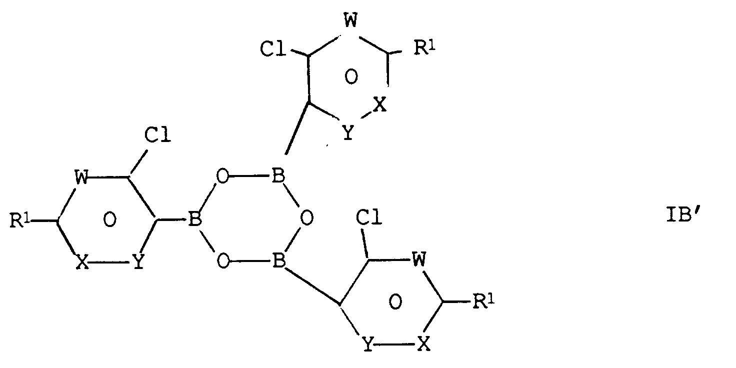
die jedoch durch Hydro- bzw. Alkolyse in die entsprechenden Verbindungen der Formel IB überführt werden.

Weiterhin ist Gegenstand der Erfindung die Verwendung der nach dem erfindungsgemäßen Verfahren hergestellten o-Chlorarylboronsäuren bzw. deren Estern der Formel IB zur Herstellung der entsprechenden o-Chlorphenole, insbesondere von 2,3-Dichlorphenol und 2,3-DiChlorhydrochinon, durch oxidative Hydrolyse, ihre Verwendung als Kopplungskomponenten bei der Übergangsmetall-katalysierten Kreuzkopplung mit Halogen- bzw. Perchloralkylsulfonverbindungen, sowie zur Herstellung der entsprechenden o-Chlorhalogenaromaten durch Halogenierung.

Die o-Chlorphenylboronsäuren bzw. deren Ester der Formel Ib sind teilweise bekannt, teilweise neu. Die neuen darunter sind ebenfalls Gegenstand der vorliegenden Erfindung, insbesondere die Verbindungen der Formeln IB1 und IB2, 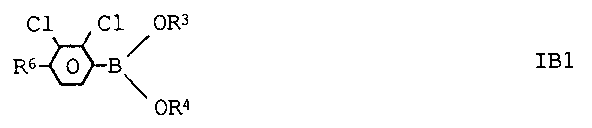
worin R³ und R⁴ die vorgegebene Bedeutung besitzen, und
- R⁶: F, Alkyl, 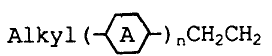 oder 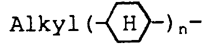 worin 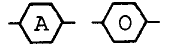 oder 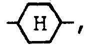 und
- n: 1 oder 2 ist, 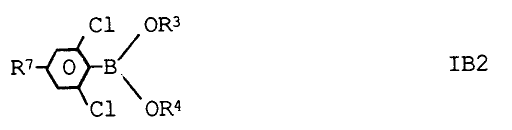
wobei R² und R³ die angegebene Bedeutung besitzen, und
- R⁷: H, F, Alkoxy mit jeweils bis zu 18 C-Atomen oder eine der Formel II entsprechende mesogene Gruppe bedeutet,
sowie deren trimere Anhydride.

Die nach dem erfindungsgemäßen Verfahren hergestellten o-Chloraryl-Derivate umfassen Mono-, Di-, Tri- und Tetra-Chlorphenyl-Derivate sowie Pentachlorphenyl-Derivate.

Daneben können nach dem erfindungsgemäßen Verfahren auch 2-Chlorpyridin-3-yl-Derivate hergestellt werden. Ob neben den Chlorsubstituenten weitere Substituenten am aromatischen Ring vorhanden sind, ist bei der Durchführung des erfindungsgemäßen Verfahrens unkritisch. Als weitere Substituenten seien beispielsweise genannt Alkyl-, Alkenyl- oder Alkoxygruppen, Halogene wie Chlor und Brom oder mesogene Gruppen genannt. Daneben können die chlorierten aromatischen Ringe auch Bestandteile von kondensierten Ringsystemen sein, wie z.B. von Naphthalinen, Di- und Tetrahydronaphthaleinen oder von 2,3,4,5-Tetrahydro-1H-3-benzazepin-Derivaten.

Der Einfachheit bedeuten im folgenden Phe eine 1,4-Phenylengruppe, worin auch eine oder zwei CH-Gruppen durch N ersetzt sein können, wobei eine 1,4-Phenylengruppe auch durch ein oder zwei Halogenatome substituiert sein kann, ArCl eine chlorierte 1,4-Phenylengruppe der Formel 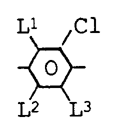
wobei L¹, L² und L³ jeweils unabhänging voneinander H oder Cl bedeuten.

Cy einen trans-1,4-Cyclohexylenrest, worin auch eine oder mehrere nicht benachbarte CH2-Gruppen durch -O- ersetzt sein können.

E bedeutet eine Gruppe, welche durch die erfindungsgemäße Reaktion eingeführt wurde.

BX₂ bedeutet einen Trioxatriborinonrest der Formel 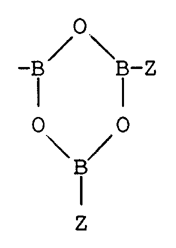
worin
- Z: die jeweils angegebene o-Fluorphenylgruppe bedeutet,
oder
eine Gruppe der Formel B(OR³)(OR⁴),
worin R³ und R⁴ die angegebene Bedeutung besitzen, vorzugsweise sind R³ und R⁴ gleich und bedeuten Wasserstoff, Methyl oder Isopropyl.

Die nach dem erfindungsgemäßen Verfahren hergestellten Verbindungen der Formel I umfassen diejenigen der Formeln Ia bis Ig 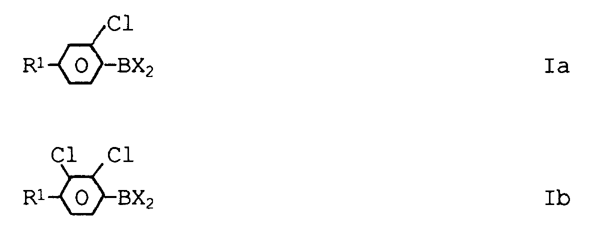 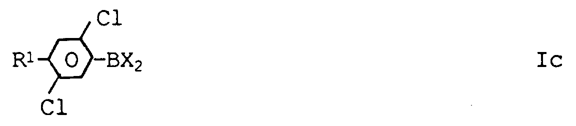
Bevorzugte Alkylierungs- bzw. Hydroxyalkylierungsreagenzien sind die Verbindungen der Formel IIIa bis IIIf: 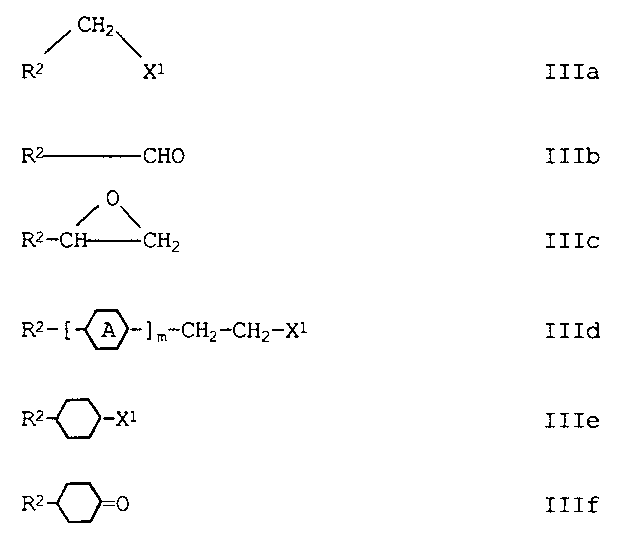
worin R² Alkyl mit 1 bis 15 C-Atome oder eine der Formel II entsprechende mesogene Gruppe bedeutet 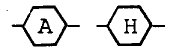
oder 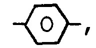
m 1 oder 2 und X¹ Cl, Br, Jod oder eine Perfluoralkylsulfonsäuregruppe bedeutet.

Silylierungsreagenzien sind die Verbindungen der Formel IIIg SI-L, worin SI die gegebene Bedeutung besitzt und L eine Abgangsgruppe darstellt, insbesondere Verbindungen der Formeln IIIga bis IIIgh:

(CH₃)₃Si-Cl IIIga

(CH₃)SiBr IIIgb

(CH₃)₃SiJ IIIgc

(CH₃)₃SiOSO₂CF₃ IIIgd

(CH₃)₂ (tert.-C₄H₉)SiCl IIIge

(C₆H₅)₂ (tert.-C₄H₉)SiCl IIIgf

(C₂H₅)₃SiCl IIIgg

(I-C₃H₇)₃SiCl IIIgh

Zur Herstellung der Verbindungen der Formel I, worin E B(OR³) (OR⁴) bedeutet, eignen sich Trialkylborate der Formel IIIh B(OR³) (OR)⁴ (OAlkyl), inbesondere B(OAlkyl)₃.

Die nach dem erfindungsgemäßen Verfahren hergestellten Verbindungen der Formel I umfassen diejenigen der Formeln Ia bis Ig 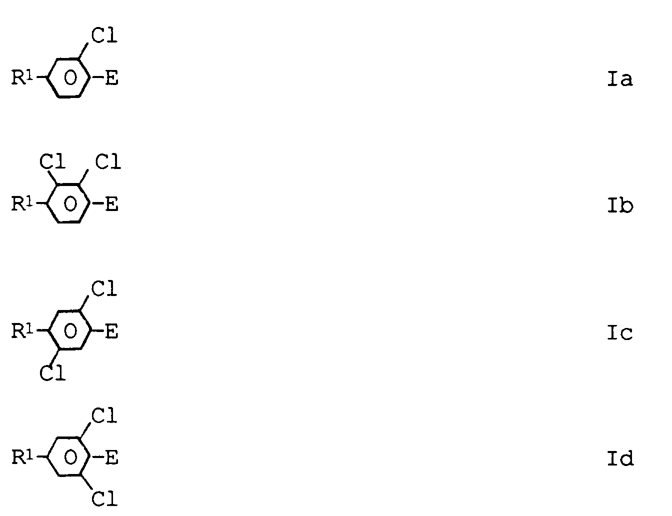 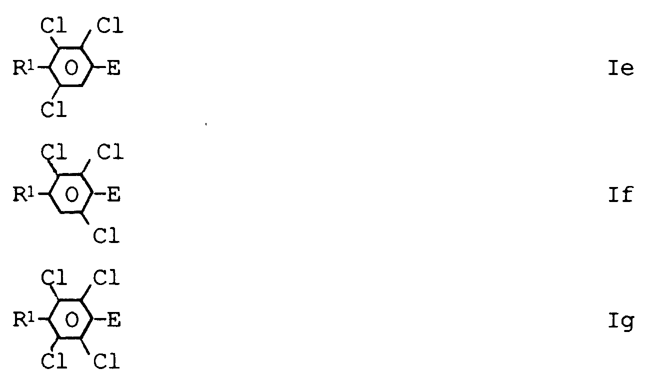
Davon sind die Verbindungen der Formeln Ia, Ib, Id und Ig besonders bevorzugt. In den genannten Verbindungen der Formeln Ia bis Ig bedeutet R¹ vorzugsweise H, Alkyl oder Alkoxy mit jeweils 1 bis 12 C-Atomen oder einem mesogenen Rest, besonders bevorzugt werden nach dem erfindungsgemäßen Verfahren die Verbindungen der Formel Ib, worin R¹ H oder Alkoxy mit 1 bis 12, insbesondere mit 2 bis 4 C-Atomen bedeutet. Diese eignen sich besonders als Zwischenprodukte zur Herstellung von Flüssigkristallen mit einem 2,3-Dichlor-1,4-phenylen bzw. 2,3-Dichlor-1,4-phenylenoxy-Struktureinheit. Die Verbindungen der Formel I, welche einen mesogenen Rest der Formel II aufweisen, umfassen demnach die Verbindungen der Formeln I1 bis I13:

R°-A¹-ArCl-E I1

R°-A¹-Z¹-ArCl-E I2

R^{o}-A¹-A²-ArCl-E I3

R^{o}-A¹-A²-Z²-ArCl-E I4

R^{o}-A¹-Z¹-A²-ArCl-E I5

R^{o}-A¹-Z¹-A²-Z²-ArCl-E I6

R^{o}-A¹-A²-A²-ArCl-E I7

R^{o}-A¹-Z¹-A²-A²-ArCl-E I8

R^{o}-A¹-A²-Z²-A²-ArCl-E I9

R^{o}-A¹-A²-A²-Z²-ArCl-E I10

R^{o}-A¹-Z¹-A²-Z²-A²-ArCl-E I11

R^{o}-A¹-Z¹-A²-A²-Z²-ArCl-E I12

R^{o}-A¹-A²-Z²-A²-Z²-ArCl-E I13

Darunter sind die Verbindungen der Formeln I1, I2, I3, I4 und I7 besonders bevorzugt.

Von den Verbindungen der Formeln I1 sind diejenigen der Formeln I1a bis I1c besonders bevorzugt.

Alkyl-Phe-ArCl-E I1a

Alkyl-Cyc-ArCl-E I1b

Alkoxy-Phe-ArCl-E I1c

Von den Verbindungen der Formel I2 sind diejenigen der Formeln I2a bis I2i besonders bevorzugt

Alkyl-Phe-CH₂CH₂-ArCl-E I2a

Alkyl-Phe-CH₂O-ArCl-E I2b

Alkyl-Phe-C≡C-ArCl-E I2c

Alkoxy-Phe-C≡C-ArCl-E I2d

Alkoxy-Phe-CH₂O-ArCl-E I2e

Alkoxy-Phe-CH₂CH₂-ArCl-E I2f

Alkyl-Cyc-CH₂CH₂-ArCl-E I2g

Alkyl-Cyc-CH₂O-ArCl-E I2h

Alkyl-Cyc-C≡C-ArCl-E I2i

In den bevorzugten Verbindungen der vor- und nachstehenden Formeln ist R¹ eine Alkylgruppe mit vorzugsweise 1 bis 10 C-Atomen, eine Alkoxy- oder eine Alkenylgruppe mit vorzugsweise jeweils 1 bis 10 C-Atomen.

Besonders bevorzugte Alkylgruppen sind Hexyl, Pentyl, Butyl, i-Butyl, Propyl, i-Propyl, Methyl und Ethyl, insbesondere Methyl; besonders bevorzugte Alkoxygruppen sind Hexoxy, Pentoxy, i-Butoxy, Propoxy, i-Propoxy, Methoxy und Ethoxy, inbesondere Methoxy; besonders bevorzugte Alkenylgruppen sind Hexenyl, Pentenyl, Butenyl und Allyl.

In den bevorzugten Verbindungen der vor- und nachstehenden Formeln können die Alkylreste, in denen auch eine CH₂-Gruppe (Alkoxy bzw. Oxaalkyl) durch ein O-Atom ersetzt sein kann, geradkettig oder verzweigt sein. Vorzugsweise haben sie 2, 3, 4, 5, 6, 7, 8, 9 oder 10 C-Atome und bedeuten demnach bevorzugt Ethyl, Propyl, Butyl, Pentyl, Hexyl, Heptyl, Octyl, Nonyl, Decyl, Propoxy, Ethoxy, Butoxy, Pentoxy, Hexoxy, Heptoxy, Octoxy, Nonoxy oder Decoxy, ferner auch Undecyl, Dodecyl, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

A¹ und A² sind bevorzugt Cyc oder Phe. In den Verbindungen der vor- und nachstehenden Formeln bedeutet Phe vorzugsweise eine 1,4-Phenylen- (Ph), eine ein- oder zweifach durch F oder CN substiuierte 1,4-Phenylengruppe (PheX) eine Pyrimidin-2,5-diyl- (Pyr), eine Pyridin-2,5-diyl- (Pyn), eine Pyrazin-3,6-diyl- oder eine Pyridazin-2,5-diyl-Gruppe, insbesondere bevorzugt Ph, PheX, Pyr oder Pyn. Vorzugsweise enthalten die nach dem erfindungsgemäßen Verfahren hergestellten Verbindungen nicht mehr als eine 1,4-Phenylengruppe, worin eine oder zwei CH-Gruppen durch N ersetzt sind. Cyc bedeutet vorzugsweise eine 1,4-Cyclohexylengruppe. Insbesondere bevorzugt sind jedoch Verbindungen der Formel I, worin eine der Gruppen A¹ und A² eine in 1- oder 4-Position durch CN substituierte 1,4-Cyclohexylengruppe bedeutet und die Nitrilgruppe sich in axialer Position befindet, d.h. die Gruppe A² bzw. A² die folgende Konfiguration aufweist: 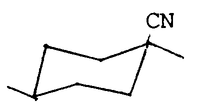
Besonders bevorzugt sind Verbindungen der Formel I und der vorstehenden Teilformeln, die eine Gruppierung -Phe-Phe- enthalten. -Phe-Phe- ist vorzugsweise -Ph-Ph-, Pyr-Phe oder Ph-Pyn. Besonders bevorzugt sind die Gruppen 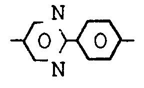
und 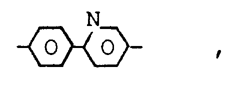
sowie ferner unsubstituiertes oder ein- oder mehrfach durch Fluor substituiertes 4,4'-Biphenylyl.

Insbesondere bevorzugt sind Verbindungen der Formel I und der nachstehenden Teilformeln, die eine 2,3-Dichlor-1,4-phenylengruppe enthalten.

Die Gruppen Z¹ und Z² bedeuten jeweils unabhängig voneinander bevorzugt eine Einfachbindung, in zweiter Linie bevorzugt -C≡C- oder -CH₂CH₂- Gruppen. Insbesondere bevorzugt sind Verbindungen der Formeln I worin eine Gruppe Z¹-CH₂CH₂-bedeutet.

Verbindungen der vor- und nachstehenden Formeln mit verzweigten Flügelgruppen R¹ können von Bedeutung sein. Verzweigte Gruppen dieser Art enthalten in der Regel nicht mehr als zwei Kettenverzweigungen. R¹ ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Der Rest R¹ kann auch ein optisch aktiver organischer Rest mit einem asymmetrischen Kohlenstoffatom sein. Vorzugsweise ist dann das asymmetrische Kohlenstoffatom mit zwei unterschiedlich substituierten C-Atomen, einem H-Atom und einem Substituenten ausgewählt aus der Gruppe Fluor, Alkyl oder Alkoxy mit jeweils 1 bis 5 C-Atomen und CN verknüpft. Der optisch aktive organische Rest R hat vorzugsweise die Formel, 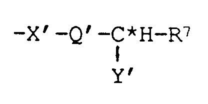
worin
- X': -O-, -S- oder eine Einfachbindung,
- Q': Alkylen mit 1 bis 5 C-Atomen, worin auch eine nicht mit X' verknüpfte CH₂-Gruppe durch -O-, ersetzt sein kann, oder eine Einfachbindung,
- Y': CN, F, CF₃, Methyl oder Methoxy, und
- R⁷: eine von Y' verschiedene Alkylgruppe mit 1 bis 15 C-Atomen, worin auch eine oder zwei nicht benachbarte CH₂-Gruppen durch -S-, -O- ersetzt sein können,
bedeutet.
- X': ist vorzugsweise eine Einfachbindung.
- Q': ist vorzugsweise -CH₂-, -CH₂CH₂-, -CH₂CH₂CH₂- oder eine Einfachbindung, insbesondere bevorzugt eine Einfachbindung.
- Y': ist vorzugsweise CH₃, -CN oder F, insbesondere bevorzugt CN oder F.
- R⁷: ist vorzugsweise geradkettiges oder verzweigtes Alkyl oder Alkoxy mit 1 bis 10, insbesondere mit 1 bis 7, C-Atomen.

Unter den Verbindungen der Formel I sowie Ia bis Ig sind diejenigen bevorzugt, in denen mindestens einer der darin enthaltenen Reste eine der angegebenen bevorzugten Bedeutungen hat.

Die nach dem erfindungsgemäßen Verfahren hergestellten Verbindungen der Formel IA 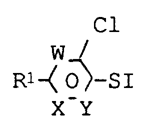
sind neu und umfassen diejenigen der Formeln IAa bis IAg 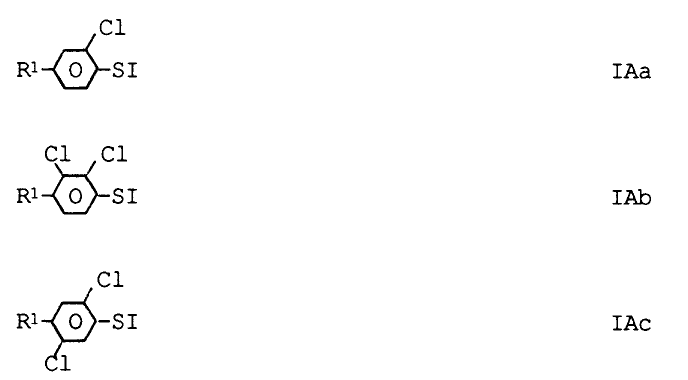 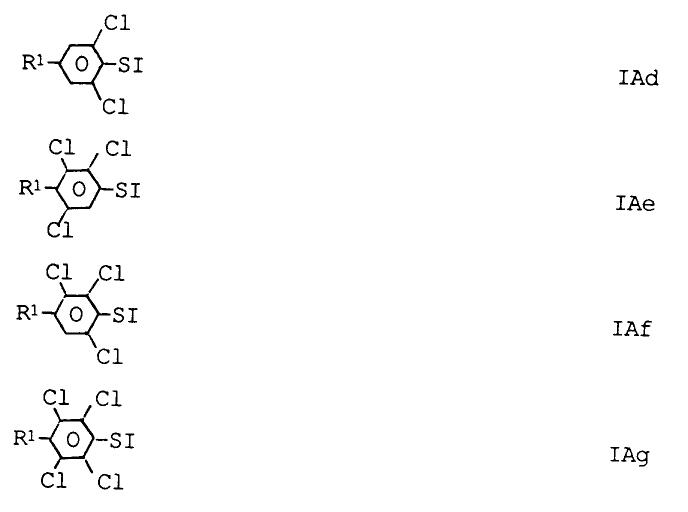
Die neuen Verbindungen der Formel IA sind ebenfalls Gegenstand der vorliegenden Erfindung. Davon sind die Verbindungen der Formeln IAa, IAb, IAd und IAg besonders bevorzugt.

Daneben eignet sich das erfindungsgemäße Verfahren zur Herstellung von neuen Dichlor-1,4-phenylendiboronsäuren bzw. deren Anhydride der Formel IB3, 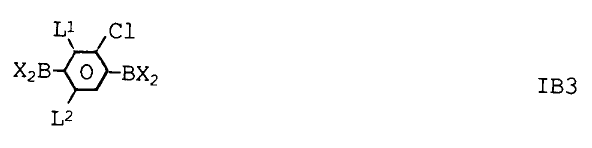
worin einer der Reste L¹, L² Cl bedeutet. Diese sind hervorragend zur Herstellung symetrischer Flüssigkristalle durch Übergangsmetall katalysierte Kreuzkopplung bzw. zur Herstellung von Dichlorhydrochinon, welches wiederum zur Synthese von Flüssigkristallen eingesetzt werden kann, (z.B. gemäß Schema I) geeignet. 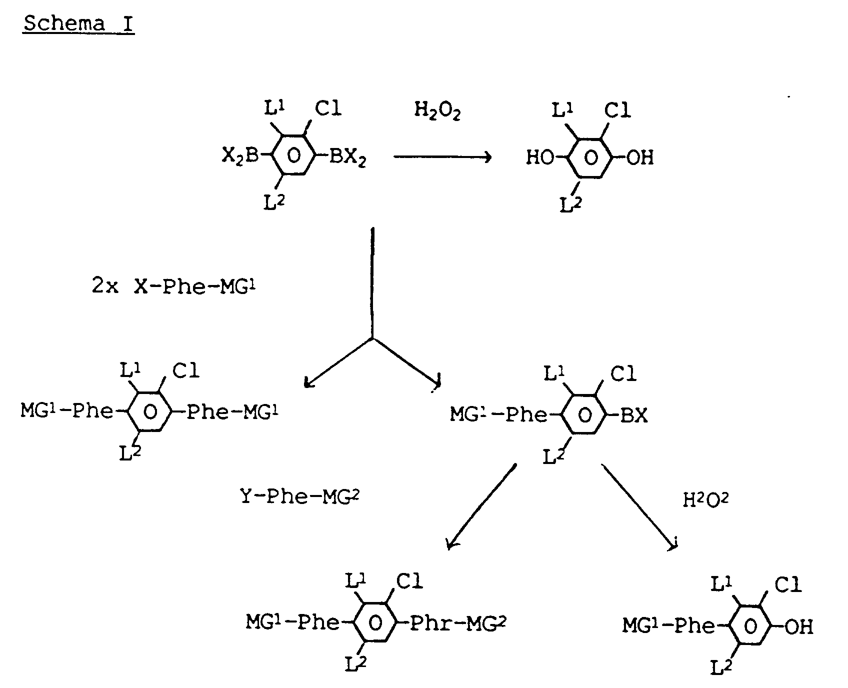
- MG¹, MG² =: mesogene Gruppen entsprechend dem Rest der Formel II
Die neuen Dichlorphenylboronsäuren der Formeln IB1 und IB2 eignen sich weiterhin zur Herstellung neuer flüssigkristalliner Dichlorphenyldioxaborinane der Formeln IB1a bzw. IB2a, 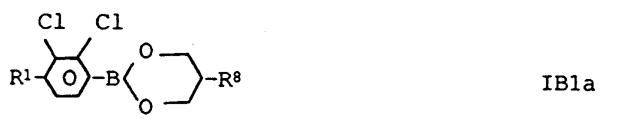 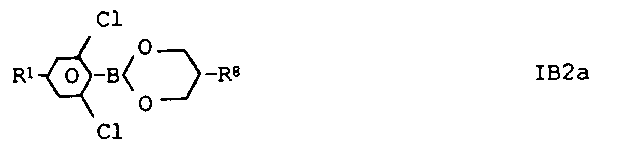
worin R¹ die angegebene Bedeutung besitzt, und
- R⁸: Alkyl, Alkenyl oder Alkoxy mit bis zu 18 C-Atomen oder eine der Formel II entsprechende mesogene Gruppe bedeutet.

Die neuen Dichlorphenyldioxanborinane der Formel IB1a und IB2a sind ebenfalls Gegenstand der Erfindung.

Die als Ausgangsstoffe benötigten Verbindungen der Formel IV, 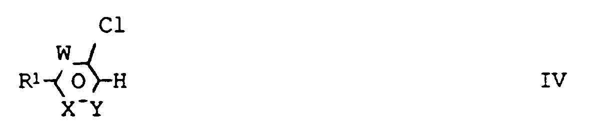
worin R¹, W, X und Y die angegebene Bedeutung besitzen, sind bekannt oder werden nach an sich bekannten Methoden, wie sie in der Literatur beschrieben sind (z.B. in den Standardwerken wie Houben-Weyl, Methoden der organischen Chemie, Georg-Thieme-Verlag, Stuttgart), und zwar unter Reaktionsbedingungen die für die genannten Umsetzungen bekannt und geeignet sind. Dabei kann man auch von an sich bekannten, hier nicht näher erwähnten Varianten Gebrauch machen.

Die Reaktionsdurchführung des erfindungsgemäßen Verfahrens ist einfach, wobei man die Ausgangsstoffe bei Temperaturen von -100° bis 100 °C, vorzugsweise -40 bis 40 °C, insbesondere 0° bis 35 °C, und bei erhöhten oder vermindertem Druck, vorzugsweise bei Normaldruck, umsetzen kann.

Ein wesentlicher Vorteil des erfindungsgemäßen Verfahrens gegenüber dem aus dem Stand der Technik bekannten ist die Tatsache, daß man nicht bei tiefen Temperaturen (-100 bis -65 °C) arbeiten muß, um eine explosionsartige Zersetzung des o-Chlorphenyllithiums bei höheren Temperaturen zu verhindern, da dieses nur in situ gebildet wird und von dem vorhandenen Alkylierungs- bzw. Hydroxyalkylierungsmittel stets abgefangen wird.

Zweckmäßigerweise legt man den chlorierten Aromaten im Gemisch mit dem Elektrophil in einem inerten Lösungsmittel vor und gibt die starke Base hinzu. Die Reaktion kann ohne oder vorteilhaft in Gegenwart eines inerten Lösungsmittels ausgeführt werden, wobei als Lösungsmittel die konventionellen Lösungsmittel für Umsetzungen mit starken Basen in Betracht kommen, z.B. Ether wie Diethylether, Tetrahydrofuran oder Methyl-tert.-Butylether, Kohlenwasserstoffe wie Pentan, Hexan, Heptan, Benzol, Toluol, Xylol oder Cyclohexan oder Gemische der genannten Lösungsmittel. Diesen Lösungsmitteln können auch Cosolventien, wie z.B. Hexamethylphosphorsäuretriamid (HMPT), Tetramethylethylendiamin (TMEDA), Dimethylpropylenharnstoff (DMPU) oder Kronenether, wie 18-Crown-6, zugesetzt werden. Die Lösungsmittelmenge ist nicht kritisch, im allgemeinen können 100 bis 1000 g Lösungsmittel je Mol chlorierter aromatischer Verbindung verwendet werden.

Als Elektrophile kommen die genannten Verbindungen der Formeln IIIa bis IIIf in Betracht, vorzugsweise n-Alkylhalogenide mit 1 bis 16 C-Atomen, insbesondere n-Alkylbromide und -jodide, wie z. B. Methyl-, Ethyl-, Propyl-, Butyl-, Pentyl-, Hexyl-, Heptyl-, Octyl- oder Nonylbromid oder Methyl-, Ethyl-, Propyl-, Butyl-, Pentyl-, Hexyl-, Heptyl-, Octyl-oder Nonyljodid, n-Alkanale mit 2 bis 16 C-Atomen, insbesondere Acetaldehyd, Propionaldehyd, Butyraldehyd, Pentanal, Hexanal, Heptanal, Octanal oder Nonanal, Oxirane wie z. B. Oxiran, 2-Methyloxiran, 2-Ethyloxiran, 2-Propyloxiran, 2-Butyloxiran, 2-Pentyloxiran, 2-Hexyloxiran oder 2-Heptyloxiran.

Aly Silylierungsmittel kommen die Verbindungen der Formeln IIIg in Betracht, vorzugsweise Trialkylsilylhalogenide wobei die Alkylgruppen geradkettig oder verzweigt sind und 1 bis 8 C-Atome aufweisen, insbesondere die Verbindungen der Formeln IIIga bis IIIgf.

Als Trialkylborate kommen üblicherweise Verbindungen der Formel B(OR³)₂(OR⁴), vorzugsweise B(OR³)₃, wobei R³ Methyl, Ethyl, Propyl, Butyl oder Isopropyl, insbesondere Methyl oder Isopropyl bedeutet, in Betracht.

Die Art der einzusetzenden starken Base richtet sich nach den eingesetzten fluorierten Aromaten. Üblicherweise werden die in der organischen Chemie gebräuchlichen starken Basen verwendet (z.B. House: Modern Synthetic Reactions 2nd Ed., Benjamin 1972, S. 547). Besonders geeignete starke Basen sind Alkalimetalle wie Lithium, Natrium oder Kalium, Alkalimetallhydride wie Lithium-, Natrium- oder Kaliumhydrid, Erdalkalimetallhydride wie Calciumhydrid, metallorganische Verbindungen, wie n-Butyllithium, sec.-Butyllithium, tert.-Butyllithium, Methyllithium, Ethyllithium oder Phenyllithium, insbesondere n-Butyllithium, starke Amidbasen wie Natriumamid, Kaliumamid, Lithiumdiisopropylamid, Lithiumcyclohexyl-isopropylamid, Lithiumdicyclohexylamid, 2,2,6,6-Tetramethylpiperidin-1-yllithium, Lithiumhexamethyldisilazan oder Kaliumhexamethyldisilazan, insbesondere Lithiumdiisopropylamid.

Ein weiterer Vorteil des erfindungsgemäßen Verfahrens liegt darin, daß es jederzeit gefahrlos unterbrochen und später wieder aufgenommen werden kann, da bei Unterbrechung nur nicht mitein ander reagierende Verbindungen, wie der chlorierte Aromat, das Alkylierungs- bzw. Hydroxyalkylierungsmittel, die protonierte Base und das o-Chloraryl-Derivat vorliegen.

In einer bevorzugten Ausführungsform des erfindungsgemäßen Verfahrens legt man den Chloraromaten zusammen mit etwa 10-80 %, insbesondere 15-25 %, des einzusetzenden Elektrophils in einem inerten Lösungsmittel, vorzugsweise Tetrahydrofuran, vor und gibt unter Inertgasatmosphäre gleichzeitig die Base, vorzugsweise Lithiumdiisopropylamid, in einem inerten Lösungsmittel zusammen mit der restlichen Menge des Elektrophils (20-90 %, vorzugsweise 75 bis 85 %) hinzu.

In der Regel benötigt man auf 1 Mol des zu deprotonierenden Chloraromaten 0,8 bis 2,2 Mol, vorzugsweise 1,2 bis 1,8 Mol Base und 0,8 bis 1,5 Mol, vorzugsweise 1,0 bis 1,3 Mol Elektrophil.

Die Aufarbeitung des Reaktionsgemisches und die Isolierung der Produkte erfolgt in üblicher Weise, z.B. indem man das Reaktionsgemisch auf Wasser und/oder Eis bzw. verdünnte Säure gießt und nach Abtrennen der wässrigen Phase die o-Fluorarylboronsäuren-Derivat durch Destillation oder Kristallisation gewinnt.

Sowohl die trimeren Anhydride der o-Chlorarylboronsäuren als auch die freien Boronsäuren können jedoch auch ohne Reinigungsschritt durch Umsetzung mit H₂O₂ zu den entsprechenden o-Fluorphenolen hydrolysiert werden.

Nach dem erfindungsgemäßen Verfahren ist es überraschend möglich, die o-Chloraryl-Derivate, die wertvolle Zwischenprodukte beispielsweise für Flüssigkristalle, Hilfsstoffe, Pflanzenschutzmittel und Pharmaka sind, in gegenüber dem Stand der Technik einfacher Art, gefahrlos, in größerem Maßstab und in höheren Ausbeuten herzustellen.

Aus den erfindungsgemäßen chlorierten Arylsilanen der Formel IV lassen sich z.B. gemäß Schema 1 o-chlorierte Phenole und Phenylboronsäuren, welche durch übergangsmetallkatalysierte Kreuzkopplung gemäß WO 89/2425 zu flüssigkristallinen Produkten umgesetzt werden können, erhalten. 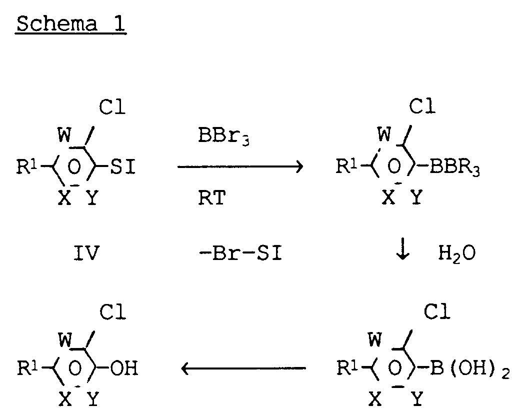
Weiterhin lassen sich aus den erfindungsgemäßen Arylsilanen der Formel IA o-chlorierte Halogenbenzolderivate gemäß Schema 2 herstellen: 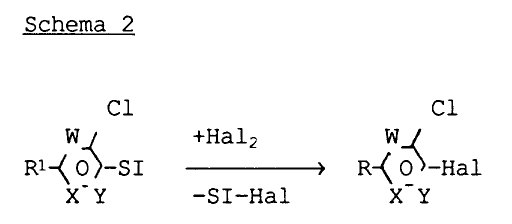
- Hal =: Br, J
Darüber hinaus lassen sich aus den erfindungsgemäßen Verbindungen der Formel IA o-chlorierte Alkyl- bzw. Acylbenzolderivate gemäß Schema 3 herstellen: 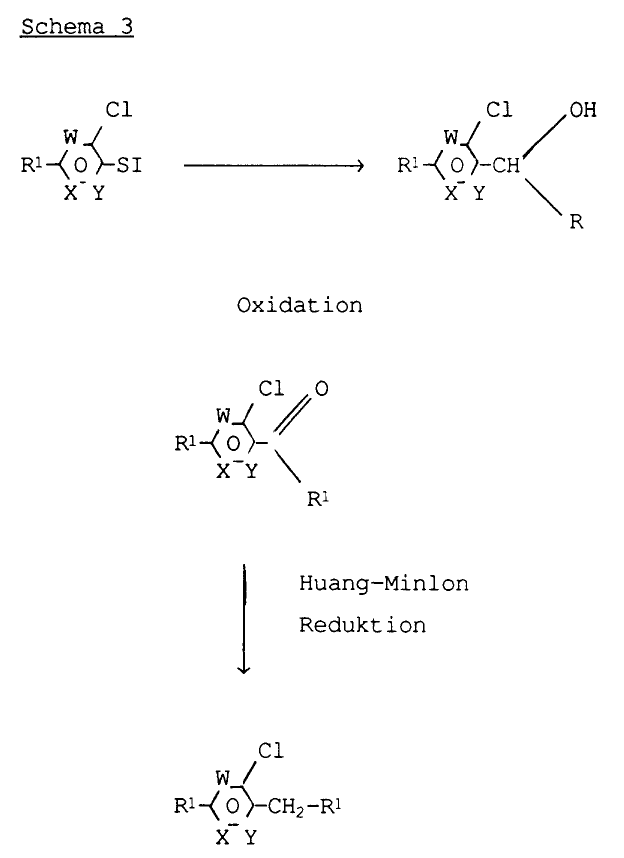
Die ¹H-Kernresonanzspektren sind aufgenommen mit einem 200 MHz Spektrometer der Fa. Bruker.

### Beispiel 1

Einsatz:
14,7 g 1,2-Dichlorbenzol 100 mmol
11,4 g Trimethylborat (TMB) 110 mmol
55 ml Lithiumdiisopropylamin-Lösung (LDA) 110 mmol
50 ml Tetrahydrofuran (THF)
6,5 ml Essigsäure
3,1 ml Schwefelsäure
13,5 ml Wasserstoffperoxid 30 %
Dichlorbenzol wird in 40 ml THF vorgelegt und mit 2 ml TMB versetzt. Das restliche TMB wird mit 10 ml THF verdünnt und gleichzeitig mit der LDA-Lösung zugetropft. Die Reaktionstemperatur wurde bei 15-25 °C gehalten.

Nach einer Nachreaktionszeit von 1/2 Std. werden 6,5 ml Essigsäure zugegeben. Anschließend werden bei gleicher Temperatur unter Kühlung 3,1 ml Schwefelsäure zugegeben.

Bei 35 °C werden 13,5 ml Wasserstoffperoxid innerhalb einer halben Stunde zugetropft. Der Ansatz wird 3 Std. am Rückfluß gekocht.

Am nächsten Tag werden 50 ml Wasser und 50 ml Methyl-tert.-butyl (MTB)-Ether zugegeben und mit Salzsäure angesäuert. Die org. Phase wird mit Wasser extrahiert und im Vakuum eingeengt.

Das Rohprodukt wurde in MTB gelöst, mit Wasser versetzt und mit Natronlauge alkalisiert (pH 11), die wäßrige Phase abgetrennt und mit Wasser gewaschen. Die org. Phasen werden verworfen, die wäßrige Phase angesäuert und mit MTB-Ether extrahiert. Die org. Phase wird zum Rückstand eingeengt.

Man erhält 9 g (= 55 % der Theorie) 2,3-Dichlorphenol MS: 162, 126, 98, 63

### Beispiel 2

### Darstellung von 1-(4'-Pentylbiphenyl-4-yl)-2-(3,5-dichlor-4-propylphenyl)-ethan

Eine Lösung von Lithiumdiisoprpylamid (0,02 mol) in THF/Hexan, hergestellt aus 0,02 mol Diisopropylamin in 25 ml THF und 12,5 ml einer 1,6 molaren Lösung von n-Butyllithium in Hexan, wird bei 25 °C tropfenweise zu einem Gemisch von 0,02 mol 1-(4'-Pentylbiphenyl-4-yl)-2-(3,5-dichlorphenyl)-ethan, 0,02 mol N,N-Dimethylpropylenharnstoff, 0,02 mol 1-Jodpropan und 25 ml THF gegeben. Nach 1,5stündigen Rühren bei Raumtemperatur wird das Reaktionsgemisch auf Wasser geschüttet, die Phasen werden getrennt und die wässrige Phase mit 2 x 50 ml Methylenchlorid extrahiert. Nach Trocknen über Magnesiumsulfat, Eindampfen des Lösungsmittels und Chromatographie erhält man das reine Produkt.

Analog werden hergestellt:

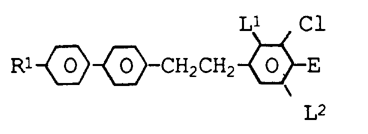

| R¹ | L¹ | L² | E |
|---|---|---|---|
| C₅H₁₁ | H | Cl | C₅H₁₁ |
| C₅H₁₁ | Cl | H | C₃H₇ |
| C₅H₁₁ | Cl | H | C₅H₁₁ |

### Beispiel 3

### Darstellung von 2-(4'-Propylbicyclohexyl-4-yl)-1-(2,6-dichlorpyridin-3-yl)-ethan

Eine Lösung von 0,02 mol Lithiumdiisopropylamid in THF/Hexan (hergestellt analog Beispiel 1) wird bei 25 °C tropfenweise zu einem Gemisch aus 0,2 mol 2,6-Dichlorpyridin, 0,02 mol N,N-Dimethylethylenharnstoff, 0,02 mol 2-(4'-Propylbicyclohexyl-4-yl)-1-jodethan, und 25 ml THF gegeben. Nach 1,5stündigen Rühren und einer Aufarbeitung wie in Beispiel 1 beschrieben erhält man das reine Produkt.

Analog werden hergestellt

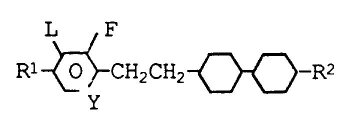

| R¹ | L | Y | R² |
|---|---|---|---|
| F₃C | H | CH | C₃H₇ |
| C₅H₁₁ | H | CCl | C₃H₇ |
| C₅H₁₁ | Cl | CH | C₃H₇ |

### Beispiel 4

### Darstellung von 2,3-Dichlortrimethylsilylbenzol

12,5 ml einer 1,6 molaren Lösung von n-Buyllithium in Hexan wird bei 0 °C tropfenweise zu einem Gemisch von 0,02 mol 1,2-Dichlorbenzol, 0,02 mol Trimethylchlorsilan und 25 ml THF gegeben. Nach 1,5stündigem Rühren bei Raumtemperatur wird das Reaktionsgemisch auf Wasser geschüttet, die Phasen werden getrennt und die wässrige Phase mit 2 x 50 ml Methylenchlorid extrahiert. Nach Trocknen über Magnesiumsulfat, Eindampfen des Lösungsmittels und Chromatographie erhält man das reine Produkt.

Analog werden hergestellt:

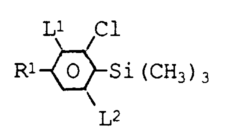

| R¹ | L¹ | L² |
|---|---|---|
| H | H | Cl |
| C₅H₁₁ | Cl | H |
| C₂H₅O | Cl | H |
| C₂H₅O | H | Cl |
| C₅H₁₁ | H | Cl |

### Beispiel 5

Aus 1,2-Dichlorbenzol, Lithiumdiisopropylamid und Trimethylborat werden analog Beispiel 1 10 g trimeres 2,3-Dichlorphenylboronsäureanhydrid hergestellt. Das Anhydrid wird in 200 ml kochendem Wasser gelöst. Man filtriert die heiße Lösung und läßt langsam auf Raumtemperatur abkühlen. Nach Abtrennen des Feststoffs und Trocknen erhält man 2,3-Dichlorphenylboronsäure.

### Beispiel 6

### 4-Propyl-2,6-dichlorphenylboronsäuredimethylester

4,9 g 4-Propyl-2,6-dichlorbenzolboronsäureanhydrid (hergestellt analog Beispiel 1) werden in 100 ml Methanol gelöst, mit 0,3 g p-Toluolsulfonsäure und 5 g Molekularsieb 4 Å versetzt und 1 Stunde unter Rückfluß gekocht. Man versetzt mit 1 g basischem Aluminiumoxid, filtriert, engt das Filtrat zum Rückstand ein und erhätl 5,4 g 4-Propyl-2,6-dichlorbenzolboronsäuredimethylester

¹H-NMR (CDCl₃/TMS): δ = 6,95 (2H), 2,9 (6H) ppm

### Beispiel 7

### 1-(4-Propyl-2,6-dichlorphenyl)-2,6-dioxaborinan

4,9 g 4-Propyl-2,6-dichlorbenzolboronsäureanhydrid (hergestellt analog Beispiel 1) werden in 200 ml Toluol gelöst und mit 3 g Propandiol-1,3 sowie 0,3 g p-Toluolsulfonsäure und 5 g Molekularsieb 4 Å versetzt. Man erhitzt 3 Stunden auf 60 °C, kühlt ab und filtriert die Mischung über eine mit 20 g basischem Aluminiumoxid gefüllte chromatographiesäule. Mit Toluol wird nacheluiert. Nach Einengen der substanztragenden Fraktionen erhält man das reine Produkt.

Analog werden hergestellt:
1-(4-Propyl-2,6-dichlorphenyl)-4-ethyl-2,6-dioxaborinan
1-(4-Propyl-2,6-dichlorphenyl)-4-propyl-2,6-dioxaborinan
1-(4-Propyl-2,6-dichlorphenyl)-4-butyl-2,6-dioxaborinan
1-(4-Propyl-2,6-dichlorphenyl)-4-pentyl-2,6-dioxaborinan
1-(4-Propyl-2,6-dichlorphenyl)-4-hexyl-2,6-dioxaborinan
1-(4-Propyl-2,6-dichlorphenyl)-4-heptyl-2,6-dioxaborinan
1-(4-Pentyl-2,6-dichlorphenyl)-4-ethyl-2,6-dioxaborinan
1-(4-Pentyl-2,6-dichlorphenyl)-4-propyl-2,6-dioxaborinan
1-(4-Pentyl-2,6-dichlorphenyl)-4-butyl-2,6-dioxaborinan
1-(4-Pentyl-2,6-dichlorphenyl)-4-pentyl-2,6-dioxaborinan
1-(4-Pentyl-2,6-dichlorphenyl)-4-hexyl-2,6-dioxaborinan
1-(4-Pentyl-2,6-dichlorphenyl)-4-heptyl-2,6-dioxaborinan
1-(4-Ethoxy-2,3-dichlorphenyl)-4-ethyl-2,6-dioxaborinan
1-(4-Ethoxy-2,3-dichlorphenyl)-4-propyl-2,6-dioxaborinan
1-(4-Ethoxy-2,3-dichlorphenyl)-4-butyl-2,6-dioxaborinan
1-(4-Ethoxy-2,3-dichlorphenyl)-4-pentyl-2,6-dioxaborinan
1-(4-Ethoxy-2,3-dichlorphenyl)-4-hexyl-2,6-dioxaborinan
1-(4-Ethoxy-2,3-dichlorphenyl)-4-heptyl-2,6-dioxaborinan
1-(4-Propyl-2,3-dichlorphenyl)-4-ethyl-2,6-dioxaborinan
1-(4-Propyl-2,3-dichlorphenyl)-4-propyl-2,6-dioxaborinan
1-(4-Propyl-2,3-dichlorphenyl)-4-butyl-2,6-dioxaborinan
1-(4-Propyl-2,3-dichlorphenyl)-4-pentyl-2,6-dioxaborinan
1-(4-Propyl-2,3-dichlorphenyl)-4-hexyl-2,6-dioxaborinan
1-(4-Propyl-2,3-difluorphenyl)-4-heptyl-2,6-dioxaborinan

### Anwendungsbeispiel 1

### Darstellung von 4-Ethoxy-2,3-dichlor-4'-pentylbiphenyl

Eine Lösung von 4-Ethoxy-2,3-dichlorphenylboronsäureanhydrid (5,7 g) in Ethanol wird bei 20 °C zu einer Lösung von 3,8 g p-Pentylbrombenzol und 0,16 g Tetrakis-(triphenylphosphin)palladium (0) in einem Lösungsmittelgemisch aus Benzol (20 ml und 2M-Na₂CO₃ (20 ml) gegeben. Die Mischung wird 30 h auf 95 °C erhitzt. Nach Abkühlen wird die Mischung 1 h mit 30%igem H₂O₂ (2 ml) bei Raumtemperatur gerührt. Nach üblichem Aufarbeiten und Umkristallisation erhält man das reine Produkt.

## Patentansprüche

1. Verfahren zur Umsetzung von chlorierten Aromaten mit Elektrophilen in ortho-Stellung zum Chloratom, dadurch gekennzeichnet, daß man zu einem Gemisch aus dem chlorierten Aromaten und dem Elektrophil eine starke Base zugibt.

2. Verfahren nach Anspruch 1 zur Herstellung von chlorierten Aromaten der Formel I, 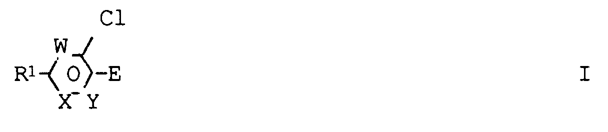 wobei
R¹ H, F, Alkyl, Alkenyl, Alkoxy mit jeweils bis zu 18 C-Atomen oder eine mesogene Gruppe,
W, X und Y jeweils unabhängig voneinander N, CH oder CCl,
und
E -CH₂-R²-, 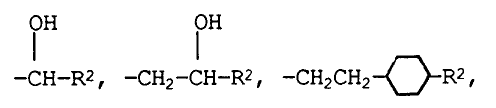 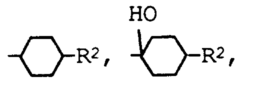 BX₂ oder SI
worin
R² Alkyl mit 1 bis 15 C-Atomen oder ein der Gruppe R¹ entsprechender mesogener Rest,
BX₂ einen Trioxatriborinonrest der Formel 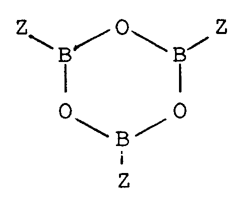 worin Z für 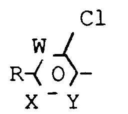 steht oder eine Gruppe der Formel -B(OR³)(OR⁴),
R³ und R⁴ H, Alkyl, Alkenyl oder Cycloalkyl mit jeweils bis zu 10 C-Atomen oder zusammengenommen eine Alkylendiylgruppe der Formel -(CH₂)ₙ- oder -CH₂CHR⁸CH₂-, worin n 2, 3 oder 4 und R⁸ Alkyl, Alkoxy oder Alkenyl mit bis zu 18 C-Atomen oder ein der Gruppe R¹ entsprechender Rest ist, und
SI eine Trihydrocarbylsilylgruppe der Formel -Si(R⁵)₃ worin R⁵ jeweils unabhängig voneinander aliphatischer, cycloaliphatischer, araliphatischer oder aromatischer Rest ist,
bedeuten.

3. Verfahren nach Anspruch 2, dadurch gekennzeichnet, daß R¹ eine mesogene Gruppe der Formel II bedeutet, wobei
R⁰-A¹-Z¹-(-A²-Z²-)-ₘ II
R⁰ einen unsubstituierten oder einen einfach durch CN, Halogen oder CF₃ substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind,
Z¹ und Z² jeweils unabhängig voneinander -CH₂CH₂-, -C≡C-, -CH₂O-, -OCH₂-, -CO-O-, -O-CO-, -CH=N-, -N=CH-, -CH₂S-, -SCH₂-, eine Einfachbindung oder eine Alkylengruppe mit 3 bis 6 C-Atomen, worin auch eine CH₂-Gruppe durch -O-, -CO-O-, -O-CO-, -CHHalogen- oder -CHCN-ersetzt sein kann,
und
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,3-Cyclobutylen, 1,3-Bicyclo(1,1,1)pentylen, 1,4-Cyclohexenylen, 1,4-Bicyclo(2,2,2)octylen, Piperidin-1,4-diyl, Naphthalin-2,6-diyl, Decahydronaphthalin-2,6-diyl und 1,2, 3,4-Tetrahydronaphthalin-2,6-diyl,
wobei die Reste (a) und (b) durch CN oder Halogen substituiert sein können, und
m 0, 1 oder 2
bedeuten.

4. Verfahren nach einem der Ansprüche 1 bis 3, dadurch gekennzeichnet, daß man als Elektrophil ein Alkylierungs- oder Hydroxyalkylierungsmittel einsetzt.

5. Verfahren nach einem der Ansprüche 1 bis 3, dadurch gekennzeichnet, daß man als Elektrophil ein Silylierungsmittel einsetzt.

6. Verfahren nach einem der Ansprüche 1 bis 3, dadurch gekennzeichnet, daß man als Elektrophil ein Trialkylborat einsetzt.

7. Verfahren nach einem der Ansprüche 1 bis 3, dadurch gekennzeichnet, daß man die Reaktion in einem inerten Lösungsmittel durchführt.

8. Verfahren nach einem der Ansprüche 1 bis 4, dadurch gekennzeichnet, daß die Reaktionstemperatur zwischen -40° und +40 °C liegt.

9. Verfahren nach einem der Ansprüche 1 bis 5, dadurch gekennzeichnet, daß man als starke Base eine metallorganische Verbindung oder ein Alkalimetallamid einsetzt.

10. Verwendung der nach einem der Ansprüche 1 bis 9 hergestellten o-Chlorarylboronsäuren bzw. deren trimeren Anhydride oder deren Estern zur Herstellung der entsprechenden o-Chlorphenolen durch oxidative Hydrolyse.

11. Verwendung der nach einem der Ansprüche 1 bis 9 hergestellten o-Chlorarylboronsäuren bzw. deren trimeren Anhydride oder deren Estern als Kopplungskomponenten bei der Übergangsmetall-katalysierten Kreuzkopplung mit Halogen- bzw. Perfluoralkylsulfonverbindungen.

12. Verwendung der nach einem der Ansprüche 1 bis 9 hergestellten o-Chlorarylboronsäuren bzw. deren trimeren Anhydride oder deren Estern zur Herstellung der entsprechenden o-Chlor-halogenaromaten durch Halogenierung.
